# EUROPÄISCHE PATENTSCHRIFT

(11) **EP 0 828 271 B1**
(45) Veröffentlichungstag und Bekanntmachung des Hinweises auf die Patenterteilung: **04.12.2002**
(21) Anmeldenummer: 96114200.7
(22) Anmeldetag: 04.09.1996
(51) Int. Cl.: H01H 21/44, A61L 9/03

(54) **Elektrische Vorrichtung zum Verdunsten von Wirkstoffen**
Electrical vaporizer for active substances
Appareil électrique de vaporisation de substances actives

(43) Veröffentlichungstag der Anmeldung: 11.03.1998
(73) Patentinhaber: Steinel GmbH & Co. KG, 33442 Herzebrock-Clarholz (DE)
(72) Erfinder: Steinel, Heinrich Wolfgang, Jr., 86825 Bad Wörishofen (DE)
(74) Vertreter: Grünecker, Kinkeldey, Stockmair & Schwanhäusser Anwaltssozietät

(56) Entgegenhaltungen:
- EP-A- 0 362 397
- DE-A- 1 665 063
- DE-A- 2 209 088
- DE-B- 1 253 791
- FR-A- 1 532 608
- GB-A- 2 285 885

## Beschreibung

Die vorliegende Erfindung bezieht sich auf eine elektrische Vorrichtung zum Verdunsten von Wirkstoffen, Parfümen od. dgl., gemäß dem Oberbegriff des Patentanspruchs 1.

Eine derartige Vorrichtung ist aus der EP 362 397 A1 bekannt geworden. Sie besitzt zum Ein- und Ausschalten der Vorrichtung einen Kippschalter mit zwei stabilen Schaltstellungen, von denen in der Regel eine eine Einschaltstellung und die andere eine Ausschaltstellung darstellt. Solche herkömmlichen Kippschalter bestehen aus einem Betätigungselement zur Betätigung eines Schaltelements, welches eine Unterbrechung und Verbindung zweier Schaltkontakte herstellt. Dabei wirkt das Betätigungselement mit einem elastischen Element zusammen, derart, daß sich zwei stabile Endstellungen für das Schaltelement ergeben und die dazwischen liegenden Stellungen instabil sind. Die oben erwähnten Bestandteile des Kippschalters sind in einem Kippschaltergehäuse montiert, wobei Anschlußfahnen von dem Gehäuse hervorstehen, die mit den Schaltkontakten verbunden sind. Zur Befestigung des Kippschalters an dem Gehäuse der Vorrichtung weist das Kippschaltergehäuse federnde Rastnasen, Vorsprünge od. dgl. auf, die in eine Öffnung in der Gehäusewand einschnappbar sind.

Es wird als nachteilig bei diesen herkömmlichen Kippschaltern angesehen, daß sie aus vielen Einzelelementen bestehen, die entweder. in mühevoller Handarbeit montiert werden müssen oder von hochkomplizierten Montagevorrichtungen zusammengebaut werden. Dies hat hohe Herstellungskosten zur Folge. Darüber hinaus ist es für den elektrischen Anschluß des Schalters erforderlich, daß Anschlußlitzen an die Anschlußfahnen des Schalters gelötet werden müssen, was zu erhöhten Herstellungskosten für die elektrischen Vorrichtungen führt, welche solche Schalter verwenden.

Weiter ist aus der EP 696 457 A1 eine elektrische Vorrichtung zum Verdunsten von Wirkstoffen bekannt mit einer im Anschlußstecker integrierten Schalteinrichtung, bestehend aus zwei Drehkupplungsteilen, die miteinander in Eingriff bringbar sind zur Herstellung einer elektrischen Verbindung zwischen den Anschlußsteckerkontakten und einem elektrischen Schaltungsteil.

Zwar entfällt bei dieser elektrischen Vorrichtung die Notwendigkeit Anschlußlitzen zu verwenden, die Herstellung der Drehkupplungsteile erfordert jedoch bei der Herstellung einigen Aufwand. Weiterhin ist die Schalteinrichtung nicht so bequem zu bedienen wie ein Kippschalter, der nur zwei stabile Schaltstellungen kennt. Vielmehr muß der Bediener die Drehkupplungsteile solange verdrehen, bis eine Raststellung gefunden ist. Daher finden elektrische Vorrichtungen mit einem Kippschalter aufgrund der leichteren Bedienbarkeit eine höhere Akzeptanz.

Die Aufgabe der vorliegenden Erfindung ist es daher, eine, einen Kippschalter aufweisende elektrische Vorrichtung der in Rede stehenden Art so weiterzubilden, daß sie einfach und mit wenig Teilen aufgebaut ist und kostengünstig herstellbar ist.

Diese Aufgabe wird von einer elektrischen Vorrichtung mit den Merkmalen des Patentanspruchs 1 gelöst.

Der Gedanke bei der erfindungsgemäßen Vorrichtung ist es, das Schaltelement und das elastische Element in Form eines einstükkigen Federblechstreifens auszubilden, wobei ein Endstück des Streifens direkt mit dem Betätigungselement gekoppelt ist und das andere Endstück an einem der Kontakte angelagert ist. Dadurch, daß der Federblechstreifen eine etwa 3/4-kreisförmige Biegung aufweist, kann er gleichzeitig die Funktion des kontaktgebenden Schaltelements und diejenige des elastischen Elements übernehmen. Dies reduziert die Anzahl der Einzelteile, aus denen der Kippschalter aufgebaut ist und gleichzeitig die Material- und Montagekosten für die elektrische Vorrichtung.

Vorzugsweise ist das eine Endstück des Federblechstreifens zwischen zwei an dem Betätigungselement angeordneten Mitnehmerzapfen gehalten. Beim Zusammenbau des Schalters kann der Federblechstreifen einfach zwischen die Mitnehmerzapfen gedrückt werden. Das Betätigungselement dient dann beim Zusammenbau des Schalters gleichzeitig als ein Montagewerkzeug.

In einer bevorzugten Ausgestaltung der elektrischen Vorrichtung weist der Federblechstreifen am anderen Endstück eine halbkreisförmige Ausnehmung auf, die in einen geeignet ausgebildeten Vorsprung des einen Kontakts eingeschnappt ist. Solche Schnappverbindungen sind schnell und einfach zu montieren.

In einer bevorzugten Ausgestaltung des Federblechstreifens sind dessen beide Enden am Auslauf der etwa 3/4-kreisförmigen Biegung um etwa 90° abgebogen.

Vorzugsweise ist das Betätigungselement mit einem daran ausgebildeten Schwenkzapfen in einer Aufnahmeöffnung in der Leiterplatine gelagert, wodurch der Kippschalter auf besonders einfache Weise direkt an der Leiterplatine anbringbar ist. Dadurch entfällt auch ein separates Kippschaltergehäuse, wie es bei den herkömmlichen Kippschaltern üblich ist.

Weiterhin ist es von Vorteil, wenn der Schwenkzapfen an seinem freien Ende einen mittigen Schlitz in Axialrichtung zur Bildung zweier halbzylindrischer Endstücke aufweist. Dadurch kann das Betätigungselement in die Aufnahmeöffnung der Leiterplatine eingeschnappt werden, was die Montage des Schalters erleichtert.

In einer bevorzugten Ausgestaltung der Erfindung ist die elektrische Leiterplatine im Gehäuse so gelagert, daß das Betätigungselement durch eine im Gehäuse ausgestaltete Öffnung teilweise hindurchtritt. Das Gehäuse wirkt dann als mechanische Führung für das schwenkbare Betätigungselement.

Vorzugsweise sind die Kontakte, die über das Schaltelement miteinander verbindbar sind, direkt auf der Leiterplatine angeordnet. Dadurch entfällt das Vorsehen von zusätzlichen Anschlußkontakten auf der Leiterplatine, die beim Stand der Technik über Litzendrähte mit den Kontakten des Kippschalters verbunden werden müssen.

Zum besseren Verständnis der vorliegenden Erfindung wird nunmehr eine bevorzugte Ausführungsform der Vorrichtung unter Bezugnahme auf die begleitenden Figuren beschrieben, welche zeigen:
Fig. 1 eine perspektivische Explosivdarstellung der erfindungsgemäßen Vorrichtung,
Fig. 2 den auf der Leiterplatine befestigten Kippschalter der Vorrichtung der Fig. 1 in der geschlossenen Stellung,
Fig. 3 den Kippschalter der Fig. 2 in einer instabilen Stellung kurz vor dem Unterbrechen der leitenden Verbindung zwischen zwei Kontakten, und
Fig. 4 den Kippschalter in der geöffneten stabilen Stellung.

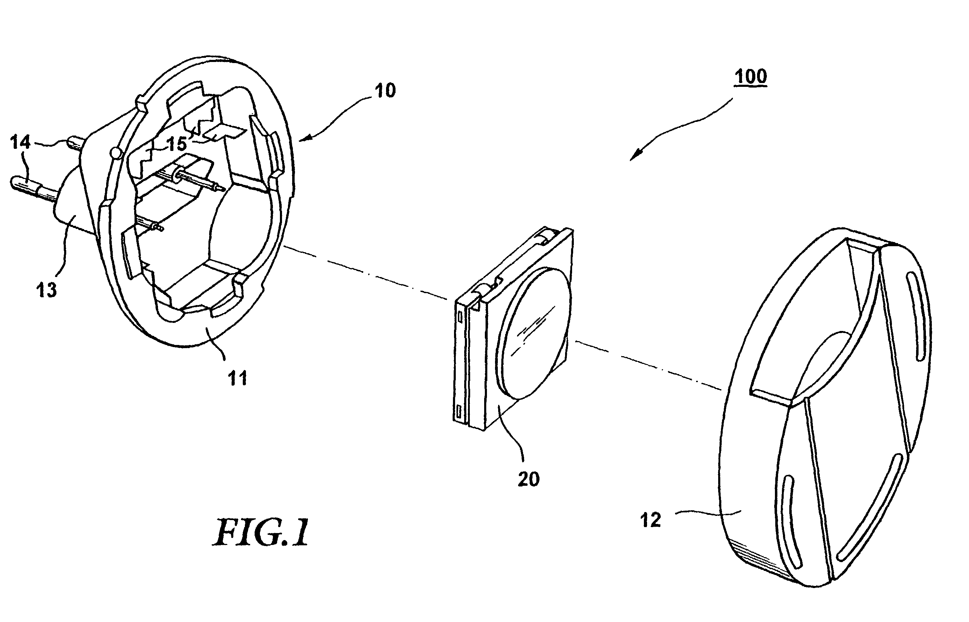

Bezugnehmend auf Fig. 1 besteht die elektrische Vorrichtung 100 aus einem zweiteiligen Gehäuse 10 und einer darin gelagerten elektrischen Leiterplatine 20 bestehend aus einem kunststoffumspritzten Stanzblechteil. Das Gehäuse umfaßt ein Anschlußteil 11 und einen daran befestigbaren Deckel 12.

Das Anschlußteil 11 besitzt an seiner rückwärtigen Außenfläche einen vorstehenden Steckersockel 13, von dem zwei Anschlußstekkerstifte 14 vorstehen. Der Steckersockel und die Anschlußstekkerstifte sind zum Anschluß an eine genormte Wand- oder Tischsteckdose geeignet geformt. Auf der Gehäuseinnenseite des Anschlußteils sind Haltestege 15 zur Halterung der elektrischen Leiterplatine 20 ausgebildet.

In den Umfangsrand des Anschlußteils 11 ist eine Öffnung 16 eingeformt, durch die ein nicht dargestellter Kippschalter teilweise durchtritt und von einem Bediener von außen betätigbar ist.

Der Deckel 12 ist mittels Rastvorsprünge an dem Anschlußteil befestigbar und weist an seinem Umfangsrand eine Öffnung zum Einführen einer nicht gezeigten Wanne aus Kunststoff oder Metall, beispielsweise Aluminium in eine im Deckel ausgebildete Tasche auf, in der sich der zu verdunstende Wirkstoff befindet. Im Deckel befinden sich auch Belüftungsöffnungen zur Luftzirkulation durch das Innere des Gehäuses.

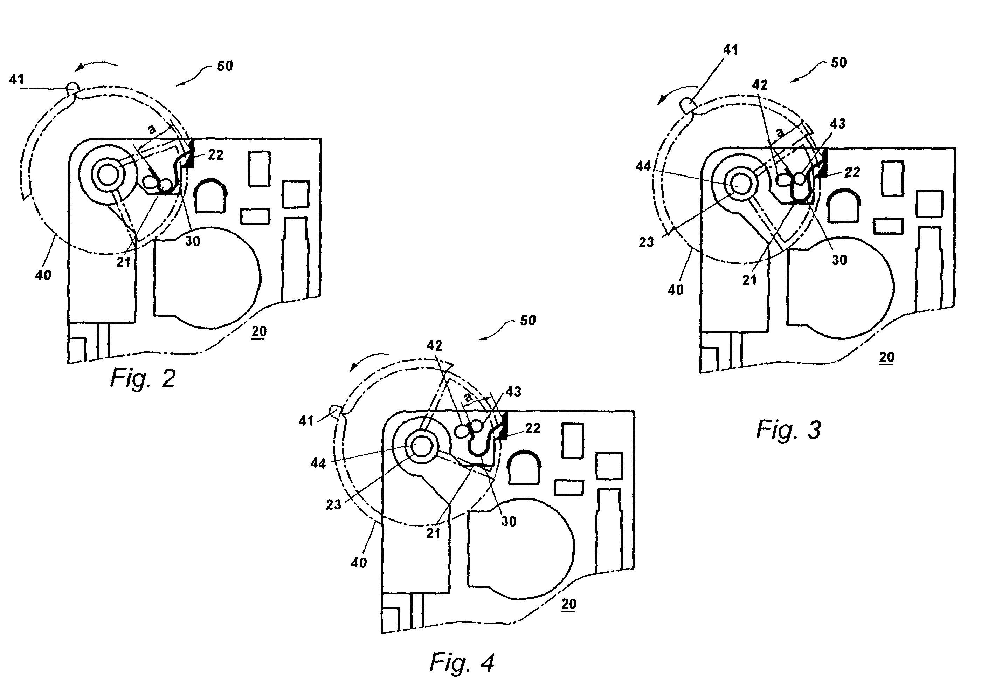

In den Fig. 2 bis 4 ist ein Kippschalter gezeigt, der allgemein mit dem Bezugszeichen 50 gekennzeichnet ist. Der Kippschalter ist an einem Eckbereich der Leiterplatine 20 montiert. Dazu ist der Eckbereich in Form einer Öse 23 ausgebildet mit einer sich seitlich daran anschließenden etwa U-förmigen Ausnehmung. Im Fußbereich und an einem Seitenschenkel der U-förmigen Ausnehmung steht jeweils ein Abschnitt eines Kontaktes 21, 22 hervor. Diese Kontakte werden im geschlossenen Zustand des Schalters elektrisch miteinander über einen Federblechstreifen 30 verbunden (Fig. 2), wogegen sie im offenen Zustand des Schalters voneinander isoliert sind (Fig. 4). Der Federblechstreifen ist so vorgeformt, daß er eine 3/4-kreisförmige Biegung aufweist, an die sich um etwa 90° abgebogene Endstücke anschließen.

Zur Betätigung des Federblechstreifens 30 ist ein Betätigungselement 40 vorgesehen, welches in etwa kreisscheibenförmig mit einem ausgebildeten Vorsprung 41 ausgebildet ist. Das Betätigungselement 41 tritt durch die Öffnung 16 im Gehäuse (Fig. 1) teilweise hindurch, wodurch es eine mechanische Führung erhält. Auf der der Leiterplatine 20 (Fig. 2-4) zugewandten Seite befindet sich ein mittig angeordneter Drehzapfen 44. Der Drehzapfen sitzt in der Öse 23 der Leiterplatine und ist in seinem Endbereich in zwei Hälften mit einem dazwischenliegenden Spalt ausgebildet, wodurch die Enden des Zapfens 44 federnd zusammendrückbar sind. Beim zusammengebauten Kippschalter ist der Drehzapfen 44 in der Öse 23 der Leiterplatine eingeschnappt.

Weiterhin befinden sich auf der der Leiterplatine 20 zugewandten Seite des Betätigungselements 40 ein innerer Mitnehmerzapfen 42 und ein dazu radial nach außen versetzter äußerer Mitnehmerzapfen 43. Zwischen den Mitnehmerzapfen befindet sich ein Spalt, der in etwa der Dicke des Federblechstreifens 30 entspricht, dessen Funktion weiter unten bei der Beschreibung des Montagevorgangs und der Bedienung des Schalters näher beschrieben werden wird.

Um die Drehung des Betätigungselements 40 um den Drehzapfen 44 auf einem Winkelbereich von ca. 30° bis 40° zu beschränken, sind sich am äußeren Umfang des Betätigungselements in axialer Richtung erstreckende Anschläge angeformt, die in den beiden stabilen Stellungen des Schalters an einer Stirnkante der Leiterplatte anliegen.

Im folgenden wird zunächst die Montage des Kippschalters 50 an der Leiterplatine der erfindungsgemäßen Vorrichtung erläutert.

Wie oben ausgeführt, ist der Kippschalter aus nur zwei Teilen gebildet, nämlich dem Betätigungselement 40 und dem Federblechstreifen 30. Der Federblechstreifen wird mit seinem einen Endstück zwischen die Mitnehmerzapfen 42, 43 des Betätigungselements eingedrückt. Der Spalt zwischen den zwei Mitnehmerzapfen ist gerade so groß, daß sich der Federblechstreifen 30 leicht klemmend dazwischenschieben läßt. Der Federblechstreifen haftet daher ohne weitere Maßnahmen an dem Betätigungselement an, welches daher gleichzeitig als Montagewerkzeug verwendet werden kann. Der Drehzapfen 44 des Betätigungselements 40 wird dann in die Öse 23 der Leiterplatine eingeschnappt. Beim Einschnappen befindet sich der Schalter in einer noch etwas weiter offenen Stellung als in Fig. 4 dargestellt, d.h. das Betätigungselement 40 ist noch etwas mehr nach links gedreht und der Federblechstreifen 30 noch nicht an dem Kontakt 22 eingerastet.

Durch Drehen des Betätigungselements 40 nach rechts wird der Federblechstreifen 30 über eine Rastnase des Kontakts 22 geschoben und verrastet dort in einer Eingriffsmulde. Durch diese einfachen Maßnahmen ist der Schalter bereits fertig montiert.

Nachfolgend soll nunmehr ein Ein- und Ausschaltvorgang der erfindungsgemäßen Vorrichtung, nämlich eine Bewegung des Kippschalters 30 zwischen seinen zwei stabilen Stellungen beschrieben werden.

Ausgehend von dem stabilen geschlossenen Zustand des Schalters, welcher in Fig. 2 gezeigt ist, wird das Betätigungselement 40 nach links gedreht und die Mitnehmerzapfen 42, 43 gleiten entlang der 3/4-kreisförmigen Biegung des Federblechstreifens nach oben. Dabei vermindert sich der Abstand a zwischen dem Spalt und dem Anlagepunkt des Federblechstreifens am Kontakt 22. Durch die Verminderung des Abstands a wird die Feder elastisch zusammengedrückt und erreicht einen instabilen Zustand (Fig. 3).

Bei einer weiteren Drehung des Betätigungselements 40 nach links entspannt sich die Feder 30 wiederum zunehmend und der Abstand a vergrößert sich (Fig. 4). Während der Drehung beschreiben die Mitnehmerzapfen 42, 43 eine etwa kreisförmige Bewegung um die Achse des Betätigungselements und heben den Federblechstreifen 30 vom Kontakt 21 ab, wodurch die elektrisch leitende Verbindung zwischen den Kontakten 21, 22 unterbrochen wird. Damit ist der stabile geöffnete Zustand des Kippschalters 50 erreicht. Eine weitere Drehbewegung des Betätigungselements 40 nach links wird durch die oben beschriebenen Anschläge verhindert.

Ein Schließvorgang des Schalters erfolgt durch Drehen des Betätigungselements nach rechts, wobei die oben beschriebenen Zustände in umgekehrter Reihenfolge eingenommen werden, was der Fachmann leicht erkennt, ohne daß dazu eine detaillierte Erläuterung von erforderlich ist.

## Patentansprüche

1. Elektrische Vorrichtung (100) zum Verdunsten von Wirkstoffen, Parfümen od. dgl., mit einem Gehäuse (10), in das ein den Wirkstoff enthaltender Behälter einsetzbar ist, mit einer im Gehäuse (10) gelagerten elektrischen Leiterplatine (20) für ein Heizelement und mit einem Kippschalter (50), der ein, um einen definierten Winkel zwischen zwei Stellungen verschwenkbares Betätigungselement (40), ein Schaltelement (30) zum Unterbrechen und Herstellen einer elektrisch leitenden Verbindung zwischen zwei Kontakten (21, 22) und ein elastisches Element aufweist, das mit dem Schaltelement und dem Betätigungselement (40) so zusammenwirkt, daß das Schaltelement zwei stabile Schaltstellungen einnimmt und dazwischen instabile Stellungen besitzt, wobei das Schaltelement und das elastische Element als ein einstückiger Federblechstreifen (30) ausgebildet sind,
**dadurch gekennzeichnet, daß**
ein Endstück des Schaltelements zwischen zwei am Betätigungselement (40) angeordneten Mitnehmerzapfen gehalten ist und das andere Endstück an einem (22) der zwei Kontakte angelagert ist und der Federblechstreifen (30) eine 3/4-kreisförmige Biegung aufweist, die durch Schwenken des Betätigungselements (40) mit dem anderen Kontakt (23) in Anlage bringbar ist.

2. Elektrische Vorrichtung nach Anspruch 1, **dadurch gekennzeichnet, daß** das andere Endstück eine halbkreisförmige Ausnehmung aufweist, die in einen Rastvorsprung des einen (22) der beiden Kontakte eingeschnappt ist.

3. Elektrische Vorrichtung nach Anspruch 1 oder 2, **dadurch gekennzeichnet, daß** die beiden Enden des Federblechstreifens (30) am Auslauf der 3/4-kreisförmigen Biegung um etwa 90° abgebogen sind.

4. Elektrische Vorrichtung nach einem der Ansprüche 1 bis 3, **dadurch gekennzeichnet, daß** das Betätigungselement (40) mit einem daran ausgebildeten Schwenkzapfen in einer Aufnahmeöffnung der Leiterplatine (20) gelagert ist.

5. Elektrische Vorrichtung nach Anspruch 4, **dadurch gekennzeichnet, daß** der Schwenkzapfen an seinem freien Ende einen mittigen Schlitz in Axialrichtung zur Bildung zweier halbzylindrischer Endstücke aufweist, die in die Aufnahmeöffnung eingeschnappt sind.

6. Elektrische Vorrichtung nach Anspruch 4 oder 5, **dadurch gekennzeichnet, daß** die elektrische Leiterplatine (20) so im Gehäuse gelagert ist, daß das Betätigungselement (40) durch eine im Gehäuse ausgebildete Öffnung teilweise durchtritt.

7. Elektrische Vorrichtung nach einem der Ansprüche 1 bis 6, **dadurch gekennzeichnet, daß** die Kontakte (21, 22), die über das Schaltelement (30) verbindbar sind, auf der Leiterplatine (20) angeordnet sind.

## Claims

1. An electric device (100) for evaporating substances, perfumes or the like, comprising a housing (10) adapted to receive a receptacle containing said substance, an electric printed circuit board (20) for a heating element, said printed circuit board being supported in said housing (10), and a toggle switch (50) including an operating element (40), which is adapted to be pivoted by a defined angle between two positions, a switching element (30) for interrupting and for establishing an electrically conductive connection between two contacts (21, 22) and a resilient element co-operating with said switching element and said operating element (40) in such a way that the switching element takes up two stable switching positions and has unstable positions between the two stable ones, said switching element and said resilient element being implemented as an integral strip-shaped sheet-metal spring (30),
**characterized in that**
one end piece of said switching element is held between two driving pins arranged on the operating element (40) and the other end piece abuts on one (22) of the two contacts, and that the strip-shaped sheet-metal spring (30) includes a ¾-circular bend which is adapted to be brought into abutment with the other contact (21) by pivoting the operating element (40).

2. An electric device according to claim 1, **characterized in that** the other end piece is provided with a semicircular opening snapped into locking engagement with a detent projection of one (22) of said two contacts.

3. An electric device according to claim 1 or 2, **characterized in that** the two ends of said strip-shaped sheet-metal spring (30) are bent off by approx. 90° at the ends of said ¾-circular bend.

4. An electric device according to one of the claims 1 to 3, **characterized in that** the operating element (40) is supported in a reception opening of the printed circuit board (20) via a pivot pin formed thereon.

5. An electric device according to claim 4, **characterized in that** a central slot extending in the axial direction is provided at the free end of said pivot pin so as to form two semicylindrical end pieces which are snapped in position in said reception opening.

6. An electric device according to claim 4 or 5, **characterized in that** the electric printed circuit board (20) is supported in the housing in such a way that the operating element (40) partially passes through an opening formed in said housing.

7. An electric device according to one of the claims 1 to 6, **characterized in that** the contacts (21, 22), which are adapted to be connected via the switching element (30), are arranged on the printed circuit board (20).

## Revendications

1. Appareil électrique (100) pour la vaporisation d'agents actifs, de parfums ou similaires, équipé d'un boîtier (10), dans lequel on peut introduire un réservoir contenant l'agent actif, d'une platine électrique à circuit imprimé (20) logée dans le boîtier (10) pour un élément individuel et d'un interrupteur à bascule (50), qui présente un élément de commande (40) pouvant basculer d'un angle défini entre deux positions, un élément de commutation (30) pour l'interruption et l'établissement d'une liaison électroconductrice entre deux contacts (21, 22) et un élément élastique, lequel agit conjointement avec l'élément de commutation et l'élément de commande (40) de telle façon que l'élément de commutation occupe deux positions de commutation stables et présente entre les deux des positions instables, l'élément de commutation et l'élément élastique étant conçus comme une bande de tôle élastique à ressorts (30) d'un seul tenant,
**caractérisé en ce qu'**une pièce d'extrémité de l'élément de commutation est maintenue entre deux tenons d'entraînement disposés sur l'élément de commande (40) et l'autre pièce est fixée sur l'un (22) des deux contacts et la bande de tôle élastique à ressorts (30) présente une partie pliée de 3/4 de cercle, qui doit être amenée en appui avec l'autre contact (23) par basculement de l'élément de commande (40).

2. Appareil électrique selon la revendication 1, **caractérisé en ce que** l'autre pièce d'extrémité est un évidement en forme de demi-cercle qui est encliqueté dans une saillie d'arrêt de l'un (22) des deux contacts.

3. Appareil électrique **caractérisé** selon la revendication 1 ou 2, **caractérisé en ce que** les deux extrémités de la bande de tôle élastique à ressorts (30) sont courbées d'environ -90° à la sortie de la partie pliée de 3/4 de cercle.

4. Appareil électrique selon l'une quelconque des revendications 1 à 3, **caractérisé en ce que** l'élément de commande (40) est logé avec un tenon pivotant formé dessus dans un orifice de logement de la platine à circuit imprimé (20).

5. Appareil électrique selon la revendication 4, **caractérisé en ce que** le tenon pivotant présente sur son extrémité libre une fente centrale dans le sens axial pour former deux pièces d'extrémité semi-cylindriques, qui sont encliquetées dans l'orifice de logement.

6. Appareil électrique selon la revendication 4 ou 5, **caractérisé en ce que** la platine électrique à circuit imprimé (20) est logée dans le boîtier de telle façon que l'élément de commande (40) traverse en partie une ouverture prévue dans le boîtier.

7. Appareil électrique selon l'une quelconque des revendications 1 à 6, **caractérisé en ce que** les contacts (21, 22), qui peuvent être reliés par l'élément de commutation (30), sont disposés sur la platine à circuit imprimé (20).
